(19) Europäisches Patentamt / European Patent Office / Office européen des brevets

(11) EP 2 522 716 B1

(12) # EUROPEAN PATENT SPECIFICATION

(45) Date of publication and mention of the grant of the patent:
**28.08.2019 Bulletin 2019/35**

(21) Application number: **11731766.9**

(22) Date of filing: **07.01.2011**

(51) Int Cl.:
***C12M 3/00*** *(2006.01)* ***C08J 7/12*** *(2006.01)*
***C08L 101/00*** *(2006.01)* ***C12M 1/00*** *(2006.01)*

(86) International application number:
**PCT/JP2011/000040**

(87) International publication number:
**WO 2011/083768 (14.07.2011 Gazette 2011/28)**

(54) **CULTURE VESSEL FOR FORMATION OF AGGREGATED CELL MASS**

KULTURGEFÄSS ZUR BILDUNG EINER AGGREGIERTEN ZELLMASSE

FLACON À CULTURE POUR FORMATION D'UNE MASSE CELLULAIRE AGRÉGÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2010 JP 2010002968**

(43) Date of publication of application:
**14.11.2012 Bulletin 2012/46**

(73) Proprietor: **Sumitomo Bakelite Company Limited**
**Shinagawa-ku**
**Tokyo 140-0002 (JP)**

(72) Inventors:
- **TSUKADA, Ryouhei**
  **Tokyo 140-0002 (JP)**
- **YOKOYAMA, Kanehisa**
  **Tokyo 140-0002 (JP)**

(74) Representative: **Müller, Christian Stefan Gerd et al**
**ZSP Patentanwälte PartG mbB**
**Hansastraße 32**
**80686 München (DE)**

(56) References cited:

| | |
|---|---|
| **JP-A- 6 327 462** | **JP-A- 7 509 405** |
| **JP-A- 11 326 311** | **JP-A- 2005 506 083** |
| **JP-A- 2006 001 995** | **JP-A- 2006 003 163** |
| **JP-A- 2006 204 232** | **JP-A- 2009 050 194** |
| **US-A- 5 665 562** | **US-A1- 2005 048 575** |

- **KENJI KAWAMURA ET AL.: 'Saibo Gyoshutai no Kochiku Gijutsu to, Saibo Kino Chosetsu Gijutsu no Kaihatsu' KYODO KENKYU TO SOKUSHIN JIGYO KENKYU SEIKA HOKOKUSHO HEISEI 9 NEN 1997, pages 233 - 242, XP008167356**

EP 2 522 716 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present invention relates to a culture vessel for forming an aggregated cell mass conveniently, the culture vessel having light-shielding property.

BACKGROUND ART

A technique for forming an aggregated cell mass has been originally developed as a culture method for maintaining high level of functionality of hepatocytes. Whereas hepatocytes in a common monolayer culture lose original liver functions within one or two days, it is known that in this method hepatocytes maintain the functions over a long period of time by forming an aggregated cell mass and further developing tissue structure when cultured under a condition that hepatocytes form an aggregated cell mass. For example, it is exemplified that an aggregated cell mass of hepatocytes cultured using proteoglycan coating maintained the liver function for two weeks or more (Non-Patent Document 1).

Then the technique for forming an aggregated cell mass has been applied to cancer cells. In this case, it is observed that, when culturing under a condition that cells form an aggregated cell mass, tissue formation is caused among cancer cells and the cells act like a cancer tissue. The technique is expected to be applied to the screening of anticancer agents (Patent Document 1).

In recent years, analysis methods in which measurement of luminescence and fluorescence of cells themselves or a component, such as a protein secreted from cells, is conducted to evaluate cell functions are becoming common.

For example, a method to measure ATP activity of aggregated cancer cells by luciferase and a method in which, after an aggregated cell mass is formed, molecules on the surface are labeled by a fluorescent substance and measurement is conducted by observing fluorescence, are commonly used.

Both cases require the following two steps.

(1) An aggregated cell mass is formed.
(2) Cell functions are measured by fluorescence and luminescence.

Currently, a commercially available plate for forming an aggregated cell mass generally utilizes a method in which the material surface is subjected to hydrophobic or super-hydrophilic treatment to suppress cell adherence to the material surface, thereby forming an aggregated cell mass by cellular interaction. Such a plate is made from a transparent resin so as to enable observation of cells. However, in analysis by luminescence and fluorescence measurements using a transparent plate, the transparency of the vessel causes leakage of luminescence and fluorescence from adjacent wells, which makes it difficult to obtain correct measured values.

Therefore, when observing luminescence and fluorescence, it is required to transfer the sample to a commercially available white or black plate. In this case, transfer of an aggregated cell mass is required. However, when the aggregated cell mass is unstable, the aggregated cell mass collapses during transfer by a pipette or dispenser, or the aggregated cell mass is adhered to a pipette or dispenser used for transfer, which are problematic. Particularly, in a case of cancer cells, many of which have poor cellular adhesiveness, an aggregated cancer cell mass falls into the state as monocellular dispersion during transfer in many cases.

Patent Document 2 describes a dual-use, high-density plate for compound storage and assays which includes a frame and a matrix of more than 384 active wells, wherein the active wells of the matrix are defined by walls disposed within the frame and bottom portions comprising a solvent resistant material.

RELATED DOCUMENTS

PATENT DOCUMENT

PATENT DOCUMENT 1: Japanese Laid-open patent publication No. 2008-22743
PATENT DOCUMENT 2: U.S. Patent Application No. 2005/0048575

NON-PATENT DOCUMENT

NON-PATENT DOCUMENT 1: N. Koide et al., Exp. Cell Res. , Vol.186, p 227(1990)

SUMMARY OF THE INVENTION

An object of the present invention is to provide a culture vessel for forming an aggregated cell mass which can form an aggregated cell mass of high quality efficiently and easily, and enables evaluation of cell functions on the spot without transferring the aggregated cell mass.

As a result of intensive study, the present inventors found that the above object can be achieved by a culture vessel having at least two wells, treating the inner surface of each of the wells so that cells cannot be adhered thereon, and making the wells from a light-shielding member, thereby completing the present invention as defined in claim 1.

Accordingly, this object can be achieved by the present invention set forth in (1) to (7) below.

(1) A culture vessel for forming an aggregated cell mass which has at least two wells,
wherein the wells are made from a light-shielding member containing a colored resin,
an inner surface including a bottom inner surface of the wells is subjected to treatment to impart cellular non-adhesiveness, and
the culture vessel is made from a transparent resin and a pigment.

(2) The culture vessel for forming an aggregated cell mass according to (1), wherein the colored resin is a white resin.

(3) The culture vessel for forming an aggregated cell mass according to (1) or (2), wherein the treatment to impart cellular non-adhesiveness for forming an aggregated cell mass is hydrophilic treatment of the surface.

(4) The culture vessel for forming an aggregated cell mass according to (3), wherein the hydrophilic treatment of the surface is conducted using the compounds represented by the following formula (Ia) or (Ib):

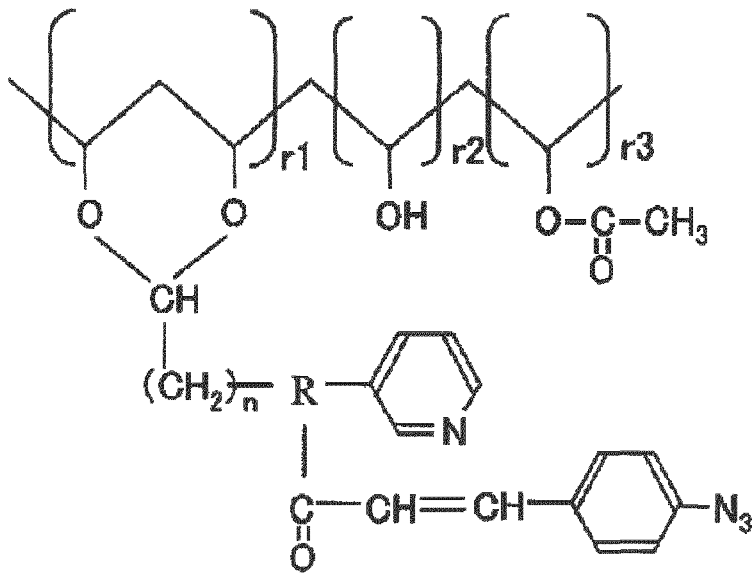

(Ia)

wherein r1 = 1 to 1,000, r2 = 40 to 4,995, r3 = 0 to 4,000, n = 1, 2 or 3 and R is an alkyl group having carbonyl and amine, and

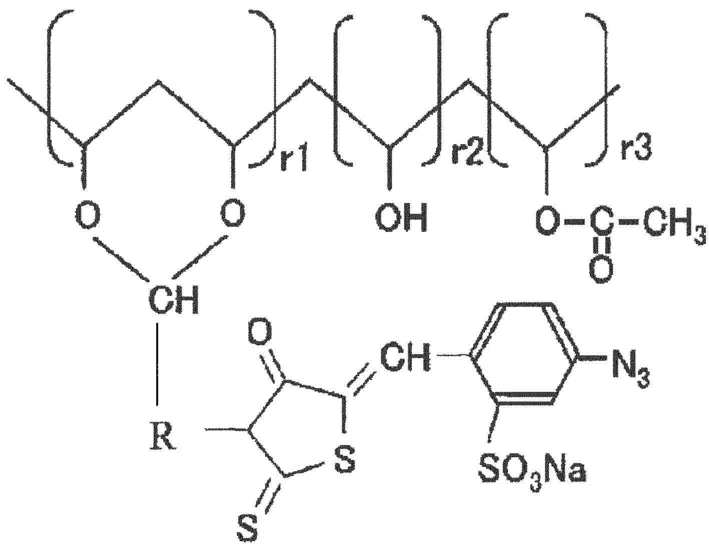

(Ib)

wherein r1 = 1 to 1,000, r2 = 40 to 4,995, r3 = 0 to 4,000 and R is an alkyl group having carbonyl and amine.

(5) The culture vessel for forming an aggregated cell mass according to any one of (1) to (4), wherein the wells have a portion in which the diameter of the inner surface of the side wall reduces toward the bottom inner surface.

(6) The culture vessel for forming an aggregated cell mass according to (5), wherein the portion in which the diameter reduces is conical or hemispheric.

(7) The culture vessel for forming an aggregated cell mass according to (3) or (4), wherein the well has a bottom part with a longitudinal section having an approximate U-shape and an approximate circular opening, at least a curved surface portion of the bottom inner surface is provided with a hydrophilic resin layer and the curvature radius (R) of the bottom inner surface is equal to or more than 1.0 mm and equal to or less than 3.0 mm.

The present invention provides a culture vessel for forming an aggregated cell mass which can form an aggregated cell mass efficiently and easily from various cells and enables evaluation of cell functions on the spot without transferring the aggregated cell mass.

BRIEF DESCRIPTION OF THE DRAWINGS

Fig. 1 shows the longitudinal section shape of the culture well.

Fig. 2 shows the longitudinal section shape of the culture well.

Fig. 3 is a graph showing the measurement results of light leakage to adjacent wells.

DESCRIPTION OF EMBODIMENTS

An important point in the culture vessel of the present invention is the following: as shown in Fig. 1(a) to (c) and Fig. 2, when using a multi-well plate in which the diameter of the side surface reduces toward the bottom surface and the portion in which the diameter reduces is hemispheric or conical, which is called a round bottom or V-bottom, one aggregated cell mass per well is formed in uniform size, which may be suitable for use in the evaluation and study of an aggregated cell mass. Fig. 1(a) shows an embodiment in which the bottom surface of the well is hemispheric, Fig. 1(b) shows an embodiment in which the bottom surface of the well is conical, and Fig. 1(c) shows an embodiment in which the bottom surface of the well is conical and the lowest part is flat. In addition, Fig. 2 shows, as described below, an embodiment, wherein the well has a bottom part with a longitudinal section being an approximate U-shape and an approximate circular opening, and in which the bottom inner surface of each of the wells has a predetermined shape. Thus, by forming the well so that the portion in which the diameter reduces is conical or hemispheric, when cells are placed in the well, the cells assemble over the vessel bottom by their own weight to easily form an aggregated cell mass.

The culture vessel of the present invention may be made from a resin material. The resin material can not only enable to make the culture vessel disposable but also form various shapes easily. Examples of the resin material include polypropylene resin, polyethylene resin, polyolefin resin such as ethylene-propylene copolymer or cyclic polyolefin resin, polystyrene, polystyrene resin such as acrylonitrile-butadiene-styrene resin, polycarbonate resin, polyethylene-terephthalate resin, methacrylic resin such as polymethyl methacrylate resin, vinyl chloride resin, polybutylene terephthalate resin, polyarylate resin, polysulfone resin, polyethersulfone resin, polyether ether ketone resin, polyether imide resin, fluorine resin such as polytetrafluoroethylene, polymethylpentene resin, acrylic resin such as polyacrylonitrile and cellulose resin such as propionate resin. Among these, polystyrene resin is especially preferable from a viewpoint of moldability and sterilizability.

When the culture vessel of the present invention is produced from the resin material, it may be produced by a method such as injection molding, blow molding or injection blow molding.

Examples of the shape of the cell culture vessel of the present invention include multi-well plates, petri dishes (dishes) and flasks. Further, sheet-like products may also be used by setting under a circumstance in which cells can be cultured such as a bottom surface of a vessel. Among these, preferred are 6 to 384-hole multi-well plates and petri dishes which are used for the preparation of a bioreactor, the evaluation of drug efficacy or a toxic substance and the development and study of artificial organs. This can improve the precision of evaluation and study using an aggregated cell mass.

As methods for shielding light between wells of a culture vessel to prevent leakage of luminescence or fluorescence to adjacent wells in order to measure a labeled substance using the luminescence or fluorescence phenomenon, a method to make a vessel from a colored resin, a method to paint a vessel with an impermeable paint or the like after making it from a transparent resin, a similar method to impart impermeability by forming a metal coating through plating or vapor deposition and the like are described herein. In the present invention, a method using a colored resin material is used.

A transparent resin may be added with a pigment and kneaded to mold. It may also be possible to mold using a molding resin material which was prepared by kneading a transparent resin with a pigment. Considering dispersibility of a pigment, it is preferable to mold using a molding resin material kneaded with a pigment.

The pigment used is not particularly specified, but it is preferable to use titanium oxide for a white vessel and carbon black for a black vessel.

In both cases, as the pigment content increases, the light-shielding property improves but the strength as a resin product decreases. Therefore, the preferred contents of titanium oxide and carbon black are 7 to 15% and 3 to 10%, respectively.

Regarding the degree of light-shielding, the lower the light transmittance to adjacent wells is, the better. The light transmittance is preferably 1% or less, more preferably 0.1% or less and most preferably 0.01% or less.

In the measurement of a labeled substance using the luminescence or fluorescence phenomenon, when placing importance on measurement sensitivity, it is preferable to use a white pigment. When using a black pigment, although the background value becomes stable, the measurement sensitivity tends to be lower than that in a case where a culture vessel using a white pigment is used and measured under the same condition. Therefore, it is more preferable to use a white pigment in the measurement of a trace amount of a sample.

Besides, a culture vessel using a white pigment has the effect that it is easier to confirm the state of an aggregated cell mass and the presence or absence of culture medium compared with a culture vessel using a black pigment.

In particular, in a culture vessel having wells shown in Fig. 2, cells assemble in a sufficient density by making the curvature radius (R') of the bottom inner surface of each well 3.0 mm or less, thereby obtaining the above-mentioned effect, which is preferable.

In addition, compared with a conventional culture vessel having a curvature radius (R') of the bottom inner surface of 3.0 mm or more, the vessel of the present invention has an excellent culture exchange efficiency (the ratio of the medium content aspirated to the total content of medium) when the aspirating medium is at the same height from the bottom part (2), since the well becomes thin toward the bottom part (2), which is also one of the key characteristics of the present invention.

Further, the vessel having the curvature radius (R') of 1.0 mm or more leads to excellent microscopic property by an inverted microscope since dead cells generated during cultivation do not aggregate too densely over the bottom surface. As a result, accurate observation of cells or an embryoid body in the well can be conducted.

Additionally, wells having 4. 0 mm or more of the diameter of the approximate circular opening (3) which leads to an excellent operability when using a multi-dispenser may be provided. 11.0 mm or less of the diameter of the approximate circular opening (3) may provide plural wells of 48 or more per culture vessel.

The volume of the well (1) is preferably equal to or more than 80 $\mu$L and equal to or less than 500 $\mu$L. This makes it possible to place a necessary and sufficient amount of medium for forming one embryoid body (EB) of cells, for example, human embryonic stem cells per well.

More preferably, the volume of the well (1) is equal to or more than 80 $\mu$L and equal to or less than 200 $\mu$L. This makes it possible to reduce the medium and reagent content used.

As shown in Fig. 2, an angle ($\theta$), formed by an intersection of a straight line (a) extending beyond the bottom part (2) of the culture well (1) from an edge line of a side surface of the culture well with a straight line (b) extending beyond the bottom part (2) of the culture well perpendicularly from the center of the opening (3), is preferably equal to

or more than 4° and equal to or less than 30°. This makes it easier for cells in medium to assemble over the bottom part (2) and also further facilitates a dispenser tip operation when exchanging medium. More preferably, the angle (θ) is equal to or more than 5° and equal to or less than 15°. This makes the approximate U-shape bottom part (2) connect to the side surface more smoothly and cells in medium are more likely to assemble, which can lead to formation of EB in a better form.

Additionally, in the present invention, the treatment to impart cellular non-adhesiveness is preferably hydrophilic treatment of the material surface. The hydrophilic treatment of the surface as used herein includes the following treatment methods.

(1) Crosslinking fixing of a water-soluble resin on an inner surface of a culture vessel
(2) Applying a hydrophilic resin on the surface

At first, method (1) will be described in detail.

This method contains the formation of a water-soluble resin covering layer by covering an inner surface including a bottom inner surface of the wells of the culture vessel with a water-soluble resin and crosslinking the resin.

A water-soluble resin as used herein means one hydrated by water molecules through ionic or hydrogen bonds to thereby be dissolved in water. In other words, a water-soluble resin is a resin having ionic or polar side chains in a necessary and sufficient amount to a main chain of a molecule for being dissolved in water. Incidentally, a water-soluble resin as used herein means one, 1.0 g or more of which is capable of being dissolved in 100 g of water at 25°C.

Examples of the water-soluble resin include saponificated polyvinyl acetate, polyvinyl pyrrolidone, polyethylene glycol, polyacrylamide, polymethacrylamide, polyhydroxyethyl methacrylate, polypentaerythritol triacrylate, polypentaerythritol tetraacrylate, polydiethylene glycol diacrylate, copolymers of monomers constituting the above and copolymers of 2-methacryloyloxyethylphosphorylcholine with other monomers (for example, butyl methacrylate and the like) . Among these, a structure containing one or more kinds selected from saponificated polyvinyl acetate, polyvinyl pyrrolidone and polyethylene glycol, and the reaction groups is preferable. This makes it possible to reduce stimuli to various cells, thereby improving a forming speed and formation rate of an aggregated cell mass as well as quality of a formed aggregated cell mass.

Saponificated polyvinyl acetate as used herein means, for example, copolymers of polyvinyl alcohol or vinyl alcohol with other compounds. Further, for example, vinyl alcohol and saponificated modified vinyl acetate which has been hydrophilic group-modified, hydrophobic group-modified, anionic modified, cationic modified, amide group-modified or reaction group-modified such as acetoacetyl group-modified are also included.

In addition, when using a polymer as the water-soluble resin, the average degree of polymerization is not particularly limited but is preferably 100 to 10,000 and especially preferably 200 to 5,000. When the average degree of polymerization is less than the lower limit, there are cases where it becomes difficult to form a uniform coating on the surface of the wells of the cell culture vessel. When the average degree of polymerization exceeds the upper limit, there are cases where the viscosity of the water-soluble resin becomes high to cause decreased workability.

When using the saponificated polyvinyl acetate, the degree of saponification of the saponificated polyvinyl acetate is not particularly limited but is preferably equal to or more than 20 mol% and equal to or less than 100 mol% and especially preferably equal to or more than 50 mol% and equal to or less than 95 mol% of the total polyvinyl acetate.

As a water-soluble resin, one containing a constitutional unit represented by the following formula (Ia) or (Ib) is preferable. This makes it possible to form a uniform coating at a practical wavelength of 300 to 500 nm, thereby reducing the amount of adhered cells and especially improving aggregated cell mass formation.

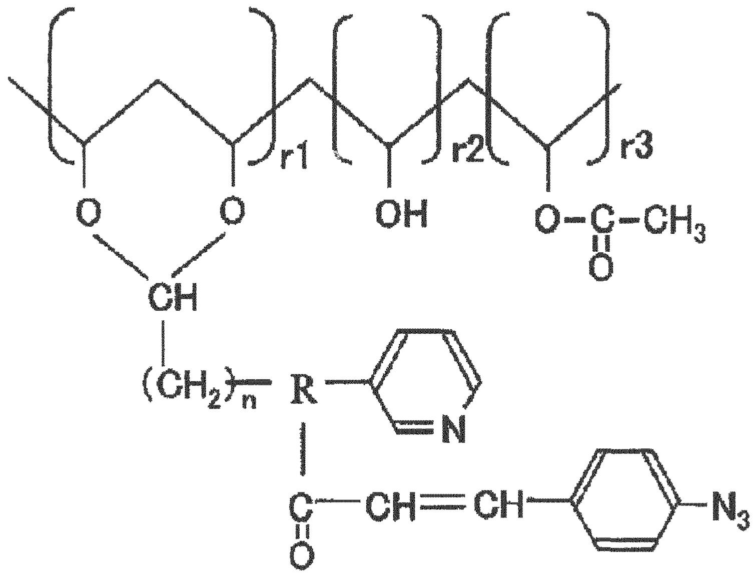

(Ia)

wherein r1 = 1 to 1,000, r2 = 40 to 4,995, r3 = 0 to 4,000, n = 1, 2 or 3 and R is an alkyl group having carbonyl and amine.

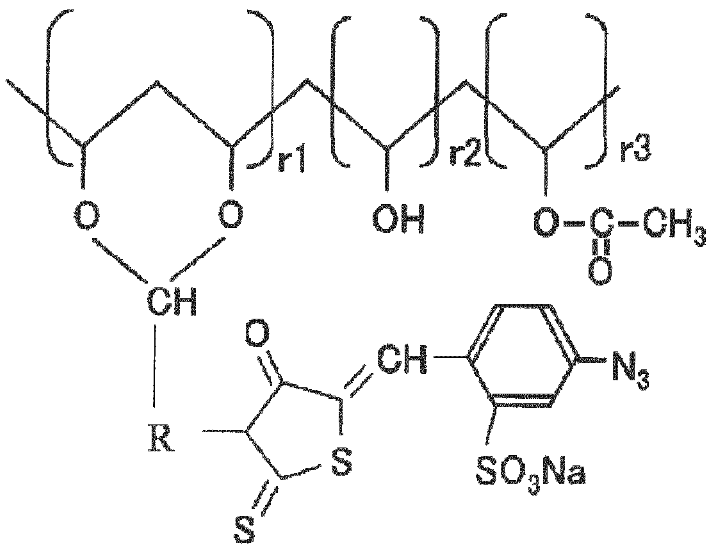

(Ib)

wherein r1 = 1 to 1,000, r2 = 40 to 4,995, r3 = 0 to 4,000 and R is an alkyl group having carbonyl and amine.

R of the formula (Ia) or (Ib) or the water-solble resin is not particularly limited as long as R is an alkyl group having carbonyl and amine but is preferably one represented by the following formula (II) . This facilitates synthesis of the polar side chains.

When immersing the cell culture vessel in a water-soluble resin, it is preferable to immerse it in a state in which the water-soluble resin is dissolved in a solvent. The solvent used thereupon may be water or a mixture of water and an organic solvent in order to increase solubility.

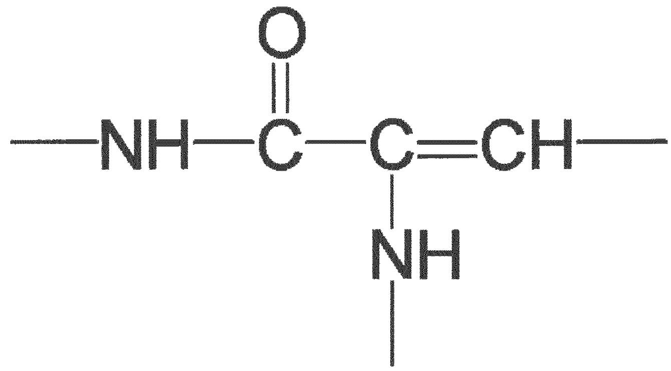

(II)

For example, when using a water-soluble resin represented by the above formula (Ia) or (Ib), usage of a 5 to 40 vol% alcohol aqueous solution as the solvent may increase solubility of the water-soluble resin, thereby forming a uniform covering layer.

The concentration of the water-soluble resin dissolved is preferably 0.01 to 30 wt% and especially preferably 0.1 to 10 wt%.

When the concentration of the water-soluble resin is too low or too high, a uniform covering layer cannot be obtained. Thus, a sufficient effect to reduce cell adhesion cannot be obtained and a good aggregated cell mass is not formed.

The thickness of the covering layer using the water-soluble resin is preferably equal to or more than 100 nm and equal to or less than 5,000 nm and more preferably equal to or more than 150 nm and equal to or less than 1,000 nm. When the thickness of the covering layer is equal to or more than the lower limit, physical stimuli which cells receive from a material can be further reduced. When the thickness is equal to or less than the upper limit, the amount of protein incorporated into the covering layer can be suppressed and cell adhesion via protein can be suppressed. Thus, the aggregated cell mass formation rate can be further improved.

As a method of covering the inner surface including a bottom inner surface of the wells of the culture vessel with the water-soluble resin, for example, spin-coating, dipping or a method in which after the water-soluble resin solution is placed in the inner surface including a bottom inner surface of the wells of the culture vessel, the solution is discharged by tilting the vessel, may be used. After bringing the water-soluble resin into contact with the inner surface of the wells of the culture vessel by these methods, the water-soluble resin solution remaining on the inner surface of the wells of the culture vessel may be dried to form a water-soluble resin covering layer.

A production method of the present invention includes a water-insoluble cured-coating modification step of curing the water-soluble resin covering layer to modify into a water-insoluble cured coating layer, after the above-mentioned step.

A surface having ionic or polar side chains in high density may be constituted by modifying the water-soluble resin covering layer into a water-insoluble cured coating layer. When coming into contact with a culture solution, the ionic or polar side chains constituted on the surface is hydrated by water molecules through electrostatic interactions or hydrogen bonds, whereby the surface of the culture vessel becomes substantially a hydrated layer with dense water molecules. This hydrated layer suppresses stimuli to cells from the material surface and an aggregated cell mass in good quality is formed rapidly. This prevents the water-soluble resin covering layer from being dissolved and released when coming into contact with a culture solution and water resistance needed as a culture vessel can be obtained.

A method of curing the water-soluble resin covering layer is not particularly limited. This can be achieved by introducing a water-soluble resin having functional groups for curing, such as radiation reactive groups, photosensitive groups or thermal reactive groups, to side chains of the water-soluble resin and curing this part. Examples of the photosensitive groups include a diazo group, azido group and cinnamoyl group, and examples of the thermal reactive groups and radiation reactive groups include a vinyl group and an epoxy group. Among these, a water-soluble resin having photosensitive groups which can be rapidly subjected to a curing treatment and can be cured using simple equipment is especially preferable.

As the photosensitive groups, a functional group having an azido group is especially preferable as shown in the formulae (Ia) and (Ib). By using this, the reaction can be performed at a practical wavelength of 230 to 500 nm and more excellent resolution can improve plasticity of the coating. Thus, a coating layer having the same thickness as that of the covering layer may be obtained by the step of forming a water-soluble resin covering layer on the surface in advance and curing the covering layer to modify into a water-insoluble cured coating layer.

Another advantage to use a water-soluble resin is that, by washing the surface with water after curing, unreacted resin can be easily washed away. When some effluent is confirmed because of poor curing reactivity and the like, by adding a washing step after curing, the effluent may be reduced and a further better formation rate of an aggregated

cell mass may be obtained.

Then, method (2) will be described.

Examples of the applied hydrophilic resin include poly-2-hydroxyethyl methacrylate (poly-HEMA), phosphorylcholine group-containing macromolecular compounds and polyethylene glycol chain-containing macromolecular compounds, but are not particularly limited thereto.

For example, a 2% ethanol solution of poly-HEMA is aliquoted in 100 μL volumes in a vessel and ethanol is evaporated to form a layer of poly-HEMA on the vessel surface. After evaporation, by washing with ultrapure water or a buffer, extra poly-HEMA molecules which do not absorb the vessel surface can be removed.

Compared with method (1), in method (2), hydrophilization of a surface is conducted only by adsorption phenomenon of a macromolecular compound on the vessel surface . Therefore the effect is weak but this method is advantageous because of its convenience.

As for the sterilization requisite for a culture vessel, for example, ethylene oxide gas sterilization, dry heat sterilization, steam sterilization, radiation sterilization and the like are exemplified. Radiation sterilization using γ ray or electron ray is preferable. In a case of mass production, γ ray sterilization is especially preferable from a viewpoint of radiolucency.

The absorbed dose of radiation is not particularly limited. However, there are cases where, when the absorbed dose is too low, sterilizability is not ensured. When the absorbed dose is too high, the cell culture vessel and the covering layer are deteriorated.

A thus-produced culture vessel has the following characteristics.

Since the material surface which has been subjected to treatment to impart cellular non-adhesiveness is conical or hemispheric, cells are likely to assemble over the bottom to easily form an aggregated cell mass.

In addition, a formed aggregated cell mass can be used for fluorescence and luminescence measurement in the same plate.

## EXAMPLES

The present invention will now be described in detail with reference to examples but the invention is not restricted thereto.

### Example 1

A 96-well multi-well plate was produced by injection molding using a resin mixture of a polystyrene resin (manufactured by PS Japan Corporation, HF77) as the resin material with a white pigment (titanium white pigment, manufactured by SUMIKA COLOR Co., Ltd.). The shape of each well was as shown in Fig. 2, the curvature radius R' was 2.0 mm, 2.6 mm and 3.2 mm and the angle θ was 85°. The obtained plate was subjected to plasma treatment (oxygen plasma, 10 min) using a plasma treatment unit (manufactured by BRANSON/IPC SERIES7000) and the plate surface was imparted with wettability as a pretreatment.

Then, polyvinyl alcohol having azido groups in side chains (manufactured by Toyo Gosei Co., Ltd. AWP: a compound of the formula (Ia) (n = 3), the average degree of polymerization of a water-soluble resin: 1600, the introduction rate of photosensitive groups: 0.65 mol%) as a water-soluble resin was dissolved in a 25 vol% ethanol aqueous solution in a polypropylene vessel light- shielded by a colored resin to prepare a 0.3 wt% solution.

To the plate, 300 μL of the water-soluble resin solution was added per well using an automatic dispenser (manufactured by BioTec Co., Ltd. Automatic Sera Washer AMW-96SII). After one-minute immersion, the plate was turned over to dispose the solution adequately and subjected to primary drying at 25°C for 17 hours, followed by radiation of UV light of 250 nm at 2.0 $mW/cm^2$ x 30 sec using a UV lamp to cure the water-soluble resin. Then, the plate was washed three times with ultrapure water, dried and then radiated by γ ray with the absorbed dose of radiation of 5.8 kGy (RADIA INDUSTRY Co., Ltd.), thereby obtaining a culture vessel (plate) of the present invention.

The following evaluations were conducted for the obtained plate.

(1) Examination of aggregated cell mass (spheroid) formation using HepG2 cells (human-hepatoma-derived cell)

HepG2 cells were dispersed in a culture solution (Dulbecco's modified MEM + 10% fetal bovine serum) at a concentration of 1 x $10^3$ cells /mL to prepare a cell-suspended solution. The solution was dispensed at 100 pL/well in the plate and cultivated at 37°C under 5% $CO_2$ atmosphere for three days. After three days, formation of an aggregated cell mass in each well was confirmed under a microscope.

Specifically, it is impossible to observe the plate by an inverted phase contrast microscope used for usual cell observation because the plate is a colored plate. Therefore, the culture vessel was placed under an optical microscope, light was cast from the upper surface and the state of cells was observed from the upper surface of the vessel, thereby

confirming formation of an aggregated cell mass in every well.

Example 2

(2) Measurement of optical transparency to adjacent wells

Evaluation of optical transparency to adjacent wells was conducted by the following method.

Chemiluminescence was carried out in a certain well and the average number of photons measured in all wells adjacent to the well in which chemiluminescence was carried out was regarded as light leakage. Light leakage was evaluated by a ratio to the number of photons measured in the well in which chemiluminescence was carried out. A specific method is the following.

An alkaline phosphatase-labeled anti-rabbit IgG antibody derived from goat (hereinafter referred to as ALP antibody for short, manufactured by Invitrogen, 65-6122) is 2000-fold diluted by a carbonate buffer (15 mM $Na_2CO_3$, 35 mM $NaHCO_3$, 0.02% $NaN_3$).

In a 4 mL-serum tube (manufactured by SUMITOMO BAKELITE CO., LTD. MS-4604W), 1 mL of the diluted solution of ALP antibody is placed, and the whole tube is covered with aluminum foil to shield light.

To the tube, 2 mL of a luminescent substrate solution, Lumiphos530 (manufactured by Lumigen, Inc. Wako Pure Chemical Industries, Ltd. 537-24662) is added and placed in a thermostatic bath at 37°C for 30 min to react ALP with a luminescent substrate. After reaction, the tube is taken out from the thermostatic bath and allowed to stand for one hour under light shielding.

200 μL of the ALP antibody-substrate reaction solution is dispensed in a well of a vessel which the background value of luminescence has been measured in advance.

Luminescence of all wells is measured with a luminescent plate reader (manufactured by Berthold Technologies, MICROLUMAT LB96D) and the light leakage is measured as transmittance.

The transmittance is determined by the following equation.

$$\text{Transmittance (\%)} = (\text{Number of photons in well in which reaction reagent is placed} - \text{Background value of number of photons})/(\text{Number of photons in adjacent well} - \text{Background value of number of photons}) \times 100$$

One example of the measurement results is shown in Fig. 3. Fig. 3 shows the measurement result in which 200 μL of the ALP antibody-substrate reaction solution was dispensed in the S-4/4 well and the number of photons was measured by luminescence. Fig. 3 shows that photons were detected also in the most adjacent wells to the well in which the reaction solution was placed and light leakage occured, although the number of photons was small. However, the light transmittance is 0.0088% at a maximum and 0.0025% at a minimum and the average is 0.0055%. Thus, it is found that light leakage is prevented.

INDUSTRIAL APPLICABILITY

Usage of the culture vessel for forming an aggregated cell mass of the present invention enables cultivation in which cell functions are maintained. Further, it can also be used for assay using functional cells.

## Claims

1. A culture vessel for forming an aggregated cell mass which has at least two wells,
wherein the wells are made from a light-shielding member containing a colored resin,
an inner surface including a bottom inner surface of the wells is subjected to treatment to impart cellular non-adhesiveness, and
the culture vessel is made from a transparent resin and a pigment.

2. The culture vessel for forming an aggregated cell mass according to claim 1, wherein the colored resin is a white resin.

3. The culture vessel for forming an aggregated cell mass according to claim 1 or 2, wherein the treatment to impart cellular non-adhesiveness for forming an aggregated cell mass is hydrophilic treatment of the surface.

4. The culture vessel for forming an aggregated cell mass according to claim 3, wherein the hydrophilic treatment of the surface is conducted using the compounds represented by the following formula (Ia) or (Ib):

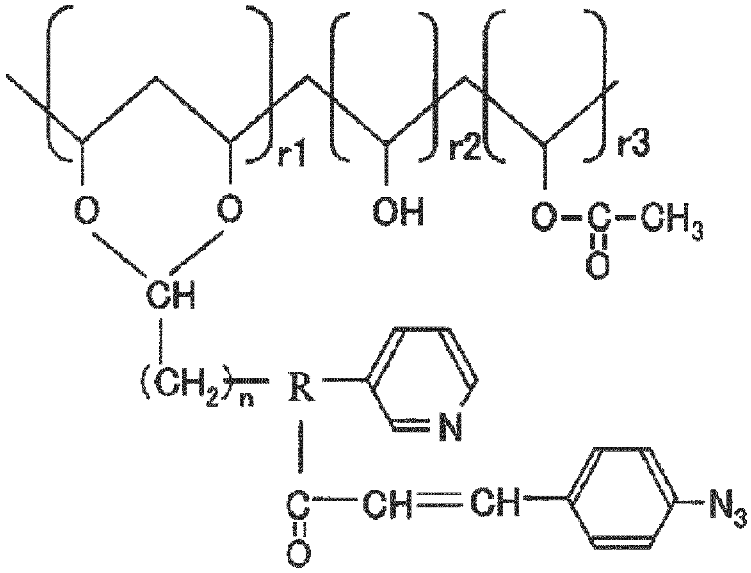

(Ia)

wherein r1 = 1 to 1,000, r2 = 40 to 4,995, r3 = 0 to 4,000, n = 1, 2 or 3 and R is an alkyl group having carbonyl and amine, and

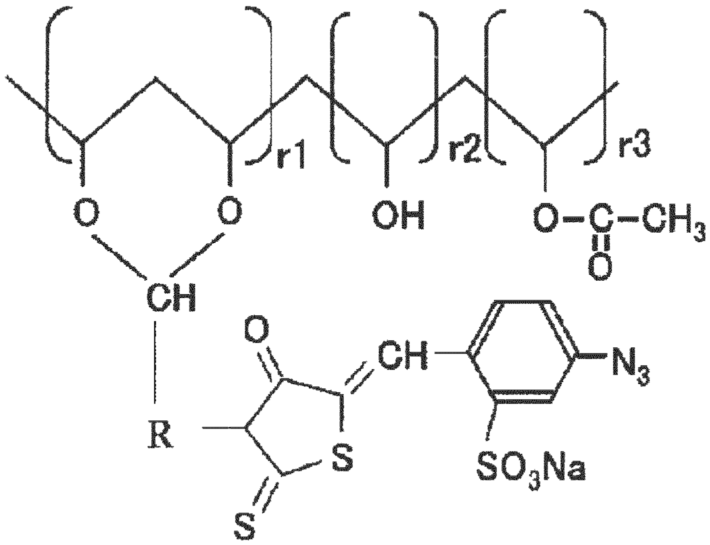

(Ib)

wherein r1 = 1 to 1,000, r2 = 40 to 4,995, r3 = 0 to 4,000 and R is an alkyl group having carbonyl and amine.

5. The culture vessel for forming an aggregated cell mass according to any one of claims 1 to 4, wherein the wells have a portion in which the diameter of the inner surface of the side wall reduces toward the bottom inner surface.

6. The culture vessel for forming an aggregated cell mass according to claim 5, wherein the portion in which the diameter reduces is conical or hemispheric.

7. The culture vessel for forming an aggregated cell mass according to claim 3 or 4, wherein the well has a bottom part with a longitudinal section having an approximate U-shape and an approximate circular opening, at least a

curved surface portion of the bottom inner surface is provided with a hydrophilic resin layer and the curvature radius (R) of the bottom inner surface is equal to or more than 1.0 mm and equal to or less than 3.0 mm.

## Patentansprüche

**1.** Kulturgefäß zum Bilden einer aggregierten Zellmasse, das mindestens zwei Vertiefungen aufweist,
wobei die Vertiefungen aus einem strahlungsabschirmenden Mitglied hergestellt sind, das ein gefärbtes Harz enthält, eine innere Oberfläche, einschließlich einer inneren Bodenoberfläche, der Vertiefungen einer Behandlung zum Verleihen von Nichthaftung von Zellen unterzogen ist und
das Kulturgefäß aus einem transparenten Harz und einem Pigment hergestellt ist.

**2.** Kulturgefäß zum Bilden einer aggregierten Zellmasse gemäß Anspruch 1, wobei das gefärbte Harz ein weißes Harz ist.

**3.** Kulturgefäß zum Bilden einer aggregierten Zellmasse gemäß Anspruch 1 oder 2, wobei die Behandlung zum Verleihen von Nichthaftung von Zellen zum Bilden einer aggregierten Zellmasse eine hydrophile Behandlung der Oberfläche ist.

**4.** Kulturgefäß zum Bilden einer aggregierten Zellmasse gemäß Anspruch 3, wobei die hydrophile Behandlung der Oberfläche unter Verwendung der Verbindungen, die durch die nachstehende Formel (Ia) oder (Ib) repräsentiert werden, durchgeführt wird:

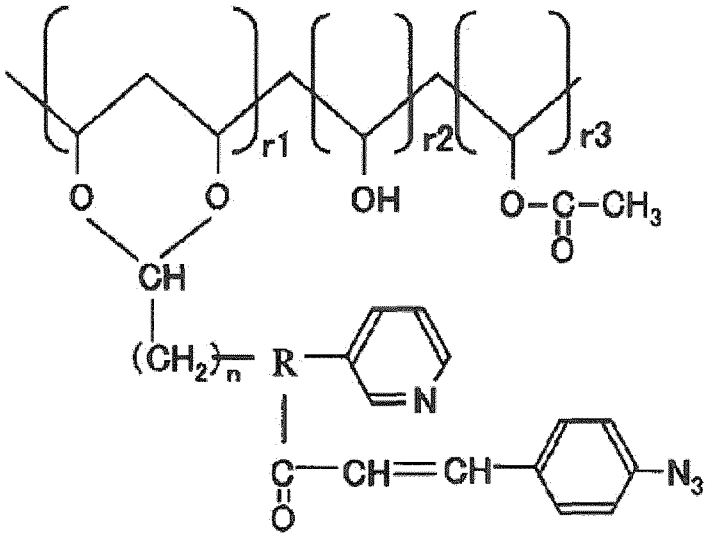

(Ia)

worin r1 1 bis 1 000 ist, r2 40 bis 4 995 ist, r3 0 bis 4 000 ist, n 1, 2 oder 3 ist und R eine Alkylgruppe mit Carbonyl- und Aminfunktionalität ist, und

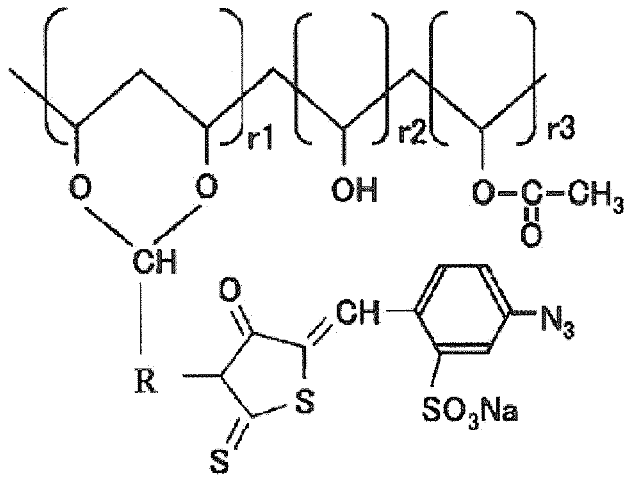

(Ib)

worin r1 1 bis 1 000 ist, r2 = 40 bis 4 995 ist, r3 0 bis 4 000 ist und R eine Alkylgruppe mit Carbonyl- und Aminfunktionalität ist.

**5.** Kulturgefäß zum Bilden einer aggregierten Zellmasse gemäß einem jeglichen der Ansprüche 1 bis 4, wobei die Vertiefungen einen Anteil aufweisen, in dem sich der Durchmesser der inneren Oberfläche der Seitenwand in Richtung der inneren Bodenoberfläche reduziert,

**6.** Kulturgefäß zum Bilden einer aggregierten Zellmasse gemäß Anspruch 5, wobei der Anteil, in dem sich der Durchmesser reduziert, konisch oder halbkugelig ist.

**7.** Kulturgefäß zum Bilden einer aggregierten Zellmasse gemäß Anspruch 3 oder 4, wobei die Wand einen Bodenanteil mit einem longitudinalen Abschnitt, der eine annähernde U-Form und eine annähernde kreisförmige Öffnung aufweist, aufweist, mindestens ein gekrümmter Oberflächenanteil der inneren Bodenoberfläche mit einer hydrophilen Harzschicht versehen ist und der Krümmungsradius (R) der inneren Bodenoberfläche gleich zu oder mehr als 1,0 mm und gleich zu oder weniger als 3,0 mm ist.

## Revendications

**1.** Cuve de culture pour former une masse cellulaire agrégée qui comporte au moins deux puits,
dans laquelle les puits sont constitués d'un élément de protection contre la lumière contenant une résine colorée, une surface interne comprenant une surface interne inférieure des puits est soumise à un traitement pour conférer une non-adhérence cellulaire, et
la cuve de culture est constituée d'une résine transparente et d'un pigment.

**2.** Cuve de culture pour former une masse cellulaire agrégée selon la revendication 1, dans laquelle la résine colorée est une résine blanche.

**3.** Cuve de culture pour former une masse cellulaire agrégée selon la revendication 1 ou 2, dans laquelle le traitement pour conférer une non-adhérence cellulaire pour former une masse cellulaire agrégée est un traitement hydrophile de la surface.

**4.** Cuve de culture pour former une masse cellulaire agrégée selon la revendication 3, dans laquelle le traitement hydrophile de la surface est conduit en utilisant les composés représentés par les formules (Ia) ou (Ib) suivantes :

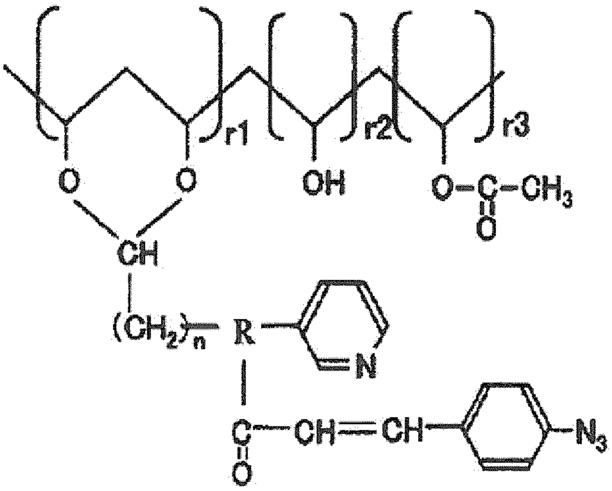

(Ia)

dans laquelle r1 = 1 à 1 000, r2 = 40 à 4 995, r3 = 0 à 4 000, n = 1, 2 ou 3 et R est un groupe alkyle comportant carbonyle et amine, et

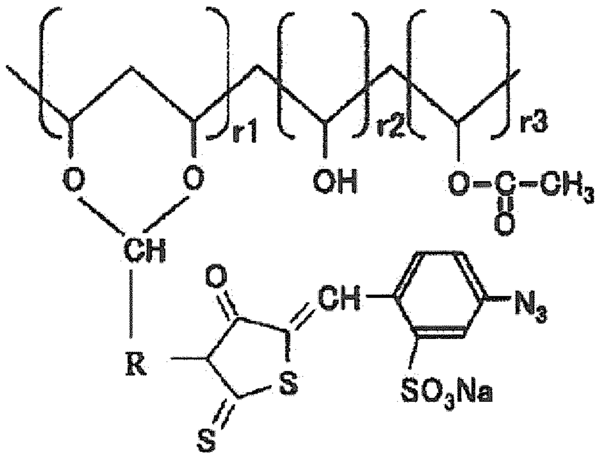

(Ib)

dans laquelle r1 = 1 à 1 000, r2 = 40 à 4 995, r3 = 0 à 4 000 et R est un groupe alkyle comportant carbonyle et amine.

**5.** Cuve de culture pour former une masse cellulaire agrégée selon l'une quelconque des revendications 1 à 4, dans laquelle les puits comportent une partie dans laquelle le diamètre de la surface interne de la paroi latérale diminue vers la surface interne inférieure.

**6.** Cuve de culture pour former une masse cellulaire agrégée selon la revendication 5, dans laquelle la partie dans laquelle le diamètre réduit est conique ou hémisphérique.

**7.** Cuve de culture pour former une masse cellulaire agrégée selon la revendication 3 ou 4, dans laquelle le puits comporte une partie inférieure avec une section longitudinale ayant approximativement une forme de U et une ouverture approximativement circulaire, au moins une partie de surface incurvée de la surface interne inférieure est pourvue d'une couche de résine hydrophile et le rayon de courbure (R) de la surface interne inférieure est égal ou supérieur à 1,0 mm et égal ou inférieur à 3,0 mm.

[Fig. 1]

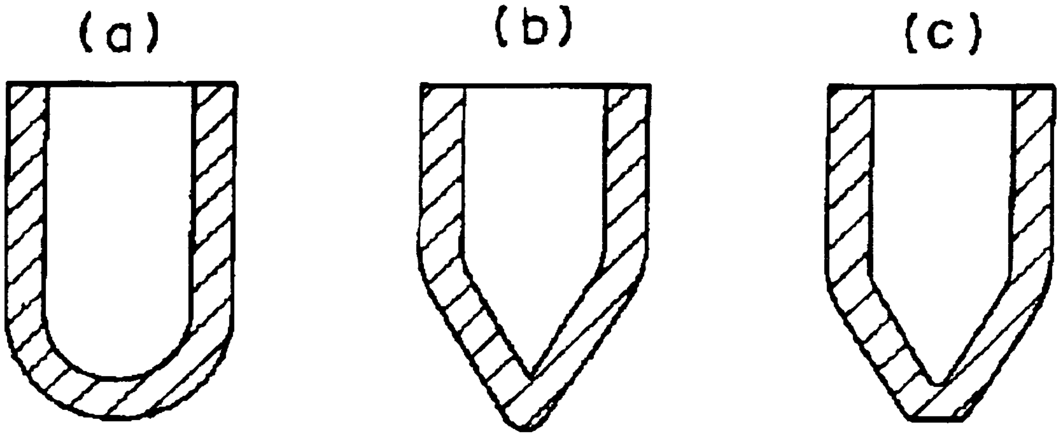


[Fig. 2]

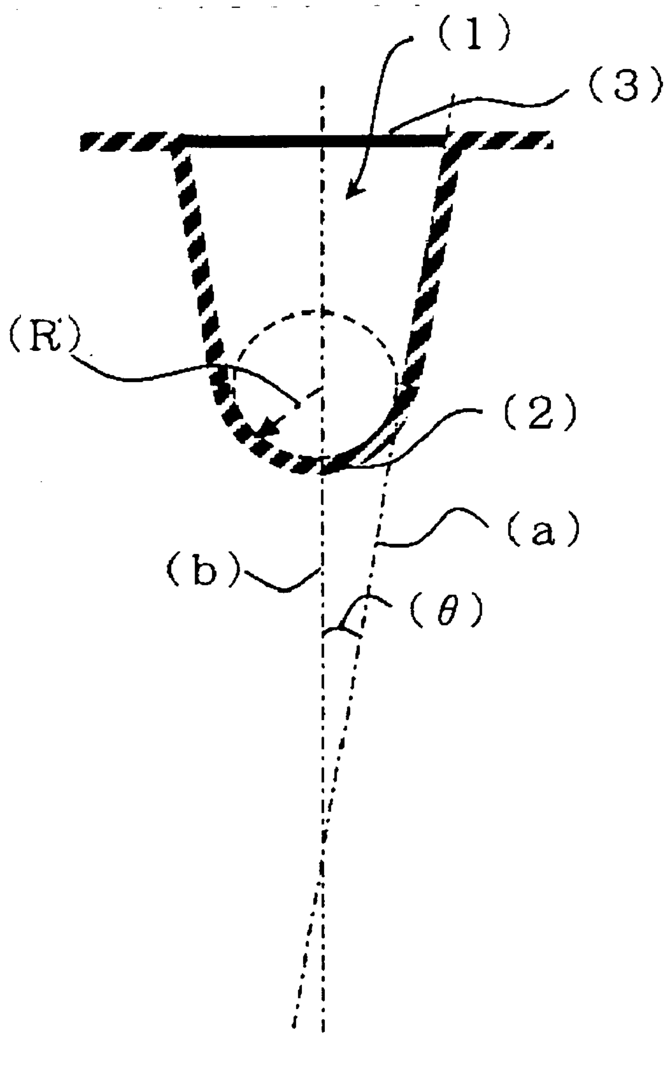

[Fig. 3]

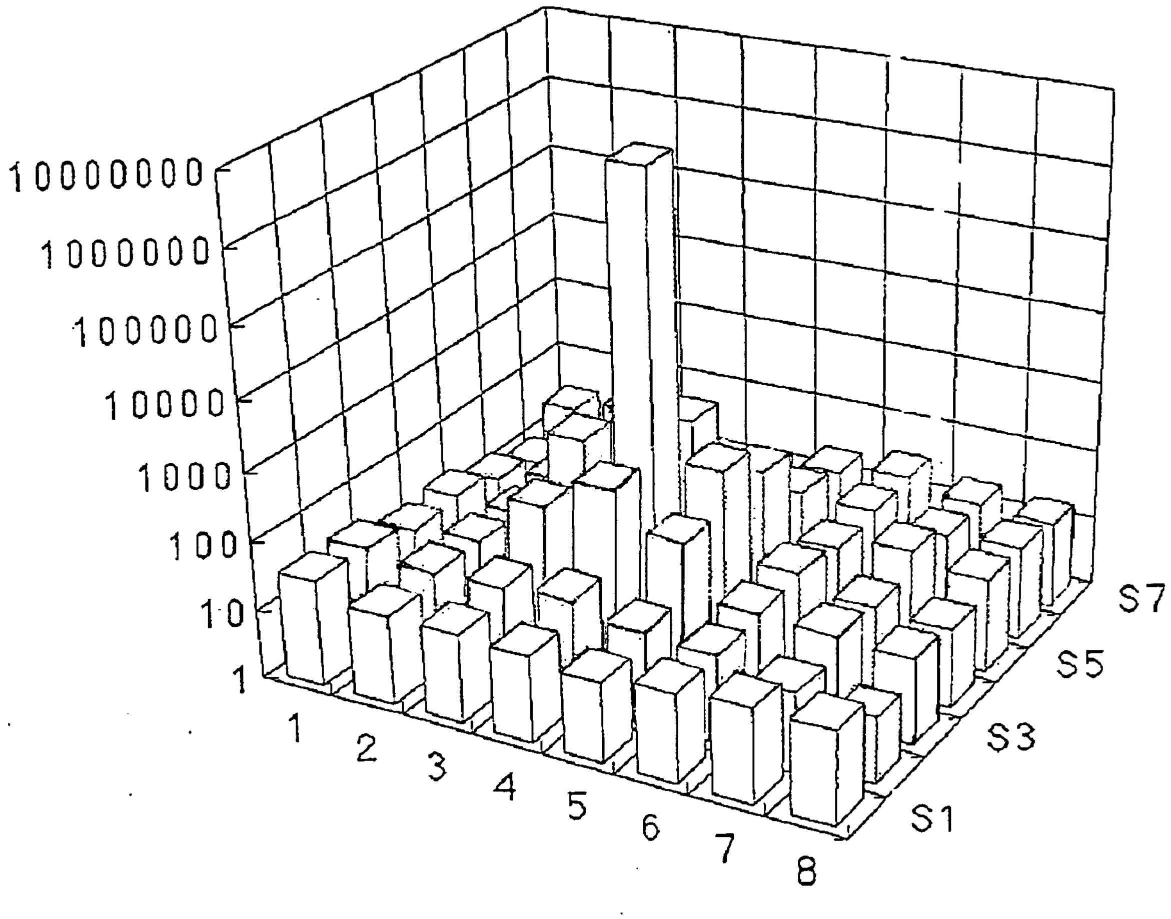
10000000
1000000
100000
10000
1000
100
10
1
1
2
3
4
5
6
7
8
S1
S3
S5
S7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008022743 A
- US 20050048575 A


### Non-patent literature cited in the description


- **N. KOIDE et al.** *Exp. Cell Res.,* 1990, vol. 186, 227